Europäisches Patentamt

European Patent Office

Office européen des brevets

(11) Publication number: **0 323 049 B1**

(12) **EUROPEAN PATENT SPECIFICATION**

(45) Date of publication of patent specification: **16.03.94**  (51) Int. Cl.⁵: **A61K 7/30**

(21) Application number: **88311431.6**

(22) Date of filing: **02.12.88**

(54) **Denture cleansing tablet.**

(30) Priority: **03.12.87 GB 8728275**

(43) Date of publication of application:
**05.07.89 Bulletin 89/27**

(45) Publication of the grant of the patent:
**16.03.94 Bulletin 94/11**

(84) Designated Contracting States:
**AT BE CH DE FR GR IT LI LU NL SE**

(56) References cited:
**EP-A- 0 151 203      EP-A- 0 248 936**
**EP-A- 0 253 772      CH-A- 465 114**
**DE-A- 2 357 720      FR-A- 2 329 747**

(73) Proprietor: **THE PROCTER & GAMBLE COM-
PANY**
**One Procter & Gamble Plaza**
**Cincinnati Ohio 45202(US)**

(72) Inventor: **Young, Kenneth**
**71 Larksfield**
**Englefield Green Surrey, TW20 0RA(GB)**

(74) Representative: **Brooks, Maxim Courtney et al**
**Procter & Gamble (NTC) Limited**
**Whitley Road**
**Longbenton**
**Newcastle-upon-Tyne NE12 9TS (GB)**

**Description**

The present invention relates to cleansing compositions in tablet form, and especially to compositions for use in cleansing dentures and the like. In particular, the invention relates to tablet-form compositions having more rapid and efficaceous denture cleansing performance and anti-bacterial activity.

Effervescent tablets for cleansing dentures and the like are well known in the art. The aim of a denture cleanser product is to clean the denture as fully and as quickly as possible and especially to remove the accummulation of plaque, mucilageneous and bacterial deposits which collect while the denture is being worn. To wear a denture which has not been completely cleaned of plaque and bacterial deposits is not only unhygienic but can also within a short space of time result in a detrimental effect on the mucous membrane. Moreover bacterial deposits can lead to so-called bacterial corrosion of the plastics material used to produce the denture with consequent color-change and malodor-formation.

Accordingly, the present invention provides a denture cleanser which cleanses more rapidly and which is more efficaceous on plaque, mucilageneous and bacterial deposits than those preparations which are presently known and available on the market.

Reference is made herein to EP-A-0253772 which discloses denture cleansers comprising bleach activator granules including a polymeric fluorocarbon binder.

According to one aspect of the invention, there is provided a cleansing tablet for dentures comprising an inorganic persalt bleaching agent, an organic peroxyacid bleach precursor and a solid base material which comprises at least one alkali metal carbonate or bicarbonate and at least one non-toxic, physiologically-acceptable organic acid and which in the presence of water releases carbon dioxide with effervescence, and wherein the organic peroxy acid bleach precursor is selected from polyacylated alkylenediamines and carboxylic esters having the general formula AcL wherein Ac is the acyl moiety of an organic carboxylic acid comprising an optionally substituted, linear or branched $C_6$-$C_{20}$ alkyl or alkenyl moiety or a $C_6$-$C_{20}$ alkyl-substituted aryl moiety and L is a leaving group, the conjugate acid of which has a pKa in the range from 4 to 13, and wherein are excluded denture cleansing tablets which additionally comprise a polymeric fluorocarbon binder.

According to another aspect of the invention, there is provided a denture cleansing tablet comprising an inorganic persalt bleaching agent, an organic peroxyacid bleach precursor and a solid base material which comprises at least one alkali metal carbonate or bicarbonate and at least one non-toxic, physiologically-acceptable organic acid and which in the presence of water releases carbon dioxide with effervescence, and wherein the organic peroxyacid bleach precursor is selected from polyacylated alkylenediamines and carboxylic esters having the general formula AcL wherein Ac is the acyl moiety of an organic carboxylic acid comprising an optionally substituted, linear or branched $C_6$-$C_{20}$ alkyl or alkenyl moiety or a $C_6$-$C_{20}$ alkyl-substituted aryl moiety and L is a leaving group, the conjugate acid of which has a pKa in the range from 4 to 13, and wherein further the inorganic bleaching agent comprises an alkali metal persulfate and the solid base material comprises at least one component phase having an acid pH in aqueous medium, said at least one component phase having incorporated therein the organic peroxy acid bleach precursor and at least a portion of the alkali metal persulfate.

All percentages and ratios herein are by weight of total composition, unless otherwise indicated.

The cleansing tablets of the invention thus comprise three essential components, a bleaching agent, a peroxyacid bleach precursor and an effervescent base composition. Each of these will be discussed in turn.

The bleaching agent takes the form of the inorganic persalt and can be selected from any of the well-known bleaching agents known for use in denture cleansers such as the alkali metal and ammonium persulfates, perborates and perphosphates and the alkali metal and alkaline earth metal peroxides. Example of suitable bleaching agents include potassium, ammonium, sodium and lithium persulfates and perborate mono- and tetrahydrates, sodium pyrophosphate peroxyhydrate and magnesium, calcium, strontium and zinc peroxides. Of these, however, the alkali metal persulfates, perborates and mixtures thereof are highly preferred for use herein.

The amount of bleaching agent in the total composition is generally from about 5 to about 70% preferably from about 10% to about 50%. In the preferred compositions comprising the mixture of alkali metal persulfates and perborates, the overall persulfate:perborate ratio is preferably from about 5:1 to about 1:5, more preferably from about 2:1 to about 1:2.

The compositions herein also contain an organic peroxyacid precursor, which in general terms can be defined as a compound having a titre of at least 1.5 ml of 0.1 N sodium thiosulfate in the following peracid formulation test.

A test solution is prepared by dissolving the following materials in 1000 mls distilled water:

| sodium pyrophosphate ($Na_4P_2O_7.IOH_2O$) | 2.5 g |
| sodium perborate ($NaBO_2.H_2O_2.3H_2O$) having 10.4% available oxygent | 0.615 g |
| sodium dodecylbenzene sulphonate | 0.5 g |

To this solution at 60°C an amount of activator is added such that for each atom of available oxygent present one molecular equivalent of activator is introduced.

The mixture obtained by addition of the activator is vigorously stirred and maintained at 60°C. After 5 minutes from addition, a 100 ml portion of the solution is withdrawn and immediately pipetted onto a mixture of 250 g cracked ice and 15 ml glacial acetic acid. Potassium iodide (0.4 g) is then added and the liberated iodine is immediately titrated with 0.1 N sodium thiosulphate with starch as indicator until the first disappearance of the blue colour. The amount of sodium thiosulphate solution used in ml is the titre of the bleach activator.

The organic peracid precursors are typically compounds containing one or more acyl groups, which are susceptible to perhydrolysis.

The activators herein are selected from:

1) Polyacylated alkylene diamines, such as N,N,N'N'-tetraacetylethylenediamine (TAED) and the corresponding methylenediamine (TAMD) and hexamethylenediamine (TAHD) derivatives, as disclosed in GB-A-907,356, GB-A-907,357 and GB-A-907,358;

2) Carboxylic esters having the general formula Ac L wherein Ac is the acyl moiety of an organic carboxylic acid comprising an optionally subsituted, linear or branched $C_6$-$C_{20}$ alkyl or alkenyl moiety or a $C_6$-$C_{20}$ alkyl-substituted aryl moiety and L is a leaving group, the conjugate acid of which has a pKa in the range from 4 to 13, for example oxybenzenesulfonate or oxybenzoate. Preferred compounds of this type are those wherein:

a) Ac is $R_3$-CO and $R_3$ is a linear or branched alkyl group containing from 6 to 20, preferably 6 to 12, more preferably 7 to 9 carbon atoms and wherein the longest linear alkyl chain extending from and including the carbonyl carbon contains from 5 to 18, preferably 5 to 10 carbon atoms, $R_3$ optionally being substituted (preferably alpha to the carbonyl moiety) by Cl, Br, $OCH_3$ or $OC_2H_5$. Examples of this class of material include sodium 3,5,5,-trimethylhexanoyloxybenzene sulfonate, sodium 3,5,5-trimethylhexanoyloxybenzoate, sodium 2-ethylhexanoyl oxybenzenesulfonate, sodium nonanoyl oxybenzene sulfonate and sodium octanoyl oxybenzenesulfonate, the acyloxy group in each instance preferably being p-subsituted;

b) Ac has the formula $R_3(AO)_mXA$ wherein $R_3$ is a linear or branched alkyl or alkylaryl group containing from 6 to 20, preferably from 6 to 15 carbon atoms in the alkyl moiety, $R_5$ being optionally subsituted by Cl, Br, $OCH_3$ or $OC_2H_5$, AO is oxyethylene or oxypropylene, m is from 0 to 100, X is O, $NR_4$ or CO-$NR_4$, and A is CO, CO-CO, $R_6$-CO, CO-$R_6$-CO or CO-$NR_4$-$R_6$-CO wherein $R_4$ is $C_1$-$C_4$ alkyl and $R_6$ is alkylene, alkenylene, arylene or alkarylene containing from 1 to 8 carbon atoms in the alkylene or alkenylene moiety. Bleach activator compounds of this type include carbonic acid derivatives of the formula $R_3(AO)_mOCOL$, succinic acid derivatives of the formula $R_3OCO(CH_2)_2COL$, glycollic acid derivatives of the formula $R_3OCH_2COL$, hydroxypropionic acid derivatives of the formula $R_3OCH_2CH_2COL$, oxalic acid derivatives of the formula $R_3OCOCOL$, maleic and fumaric acid derivatives of the formula $R_3OCOCH=CHCOL$, acyl aminocaproic acid derivatives of the formula $R_3CONR_1(CH_2)_6COL$, acyl glycine derivatives of the formula $R_3CONR_1CH_2COL$, and amino-6-oxocaproic acid derivatives of the formula $R_3N(R_1)CO(CH_2)_4COL$. In the above, m is preferably from 0 to 10, and $R_3$ is preferably $C_6$-$C_{12}$, more preferably $C_6$-$C_{10}$ alkyl when m is zero and $C_9$-$C_{15}$ when m is non-zero. The leaving group L is as defined above.

The level of peroxyacid bleach precursor by weight of the total composition is preferably from 0.1% to 10%, more preferably from 0.5% to 5%.

The effervescent solid base material of the compositions herein comprises a combination of at least one alkali metal carbonate or bi-carbonate, such as sodium bicarbonate, sodium carbonate, sodium sesquicarbonate, potassium carbonate, potassium bicarbonate, or mixtures thereof, in admixture with at least one non-toxic, physiologically-acceptable organic acid, such as tartaric, fumaric, citric, malic, maleic, gluconic, succinic, salicylic, adipic or sulphamic acid, sodium fumarate, sodium or potassium acid phosphates, betaine hydrochloride or mixtures thereof. These effervescent bases produce a vigorous effervescence when in contact with water. The (bi)carbonate components generally comprise from 5% to 65%, preferably from 25% to 55% of the total tablet; the acid components generally comprise from 5% to 50%, preferably from 20% to 40% of the total tablet.

In preferred embodiments, the solid base material comprises two or more component phases having differing pH in aqueous medium, at least one of the component phases having an acidic pH. In such compositions, the inorganic bleaching agent preferably comprises an alkali metal persulfate and the acidic phase incorporates therein the organic peroxyacid bleach precursor and at least a portion, preferably from 10% to 40%, more preferably from 15% to 30% of the alkali metal persulfate.

In highly preferred embodiments, the solid base material also comprises at least one phase having an alkaline pH. In such compositions, the inorganic bleaching agent preferably comprises both an alkali metal persulfate and an alkali metal perborate, the acidic phase incorporating therein the organic peroxyacid bleach precursor and at least a portion, preferably from about 10% to 40%, more preferably from 15% to 30% of the alkali metal persulfate, and the alkaline phase incorporating therein the alkali metal perborate.

For optimum antiplaque and antibacterial performance, the cleansing tablet is preferably designed in such a way that the alkaline phase dissolves or disperses in water more slowly or later than the acidic phase in order to provide, on placing the tablet in water, an initial pH in the acidic range, preferably from 2 to 6.5 and especially from 3 to 5. Moreover, it is preferred that the alkaline phase be present in sufficient excess of the acidic phase in order to shift, upon completion of effervescence, the pH of the aqueous medium into the alkaline range, preferably to a pH of from 7.5 to 9. The initial acidic pH should be maintained for a time from 30 seconds to 3 hours, preferably from 1 minute to 60 minutes and especially from 12 to 30 minutes.

The slower dissolution rate of the alkaline phase compared with the acidic phase can be achieved in various ways, for example by the use of alkaline salts or compounds which are inherently sparingly or slowly soluble such as anhydrous sodium carbonate, calcium carbonate, calcium hydroxide, magnesium oxide, or magnesium hydroxide carbonate; or by converting at least a portion of the alkaline phase into a state of slower dissolution rate for example by melting some of the alkaline phase components to form melt agglomerates followed by comminution to the desired particle size, or by compressing the alkaline phase components with a filler which has a slow dissolution rate such as anhydrous sodium sulfate and slowly water-soluble polymers, for example, proteins, cellulose ethers, cellulose esters, polyvinyl alcohol, alginic acid esters, vegetable fatty acid glycerides and other polymers having a colloidal or pseudo-colloidal character. Moreover, the relative rate of dissolution of the alkaline and acidic phases can be additionally controlled by appropriate formulation and distribution of the effervescent components of the solid base material. In particular, the acidic phase, at least, should incorporate both components of the carbonate or bicarbonate/acid effervescent couple and the effervescence factor of the acidic phase (i.e. the percentage of the phase represented by the least significant of the (bi)carbonate or acid components, measured on a stoichiometric basis) should be greater than that of the alkaline phase and preferably should be greater by a factor of at least 2, and more preferably at least 5. The level of (bi)carbonate and acid components in the acid phase both generally lie in the range from 10% to 50% by weight thereof, and preferably from 20% to 40% thereof. In highly preferred compositions, the alkaline phase contains a proportion of carbonate or bicarbonate (generally from 5% to 40%, preferably from 10% to 30% thereof) but is substantially free of any acidic component so that the effervescence factor of the alkaline phase as defined herein is zero.

The compositions of the invention can be in the form of monolayer tablets or can comprise two or more discrete layers. In the case of monolayer tablet, the alkaline and acidic phases are compressed, together with other optional constituents, to a tablet form using, for example, 1-5% by weight, referred to the total tablet composition, of polyvinylpyrrolidone, poly(oxyethylene) of molecular weight 20,000 to 500,000, polyethyleneglycols of molecular weight of from about 1000 to about 50,000 or Carbowax having a molecular weight of from 4000 to 20,000 as binder.

In the case of multilayer tablets, preferably at least one layer comprises the acidic phase and at least one other layer comprises the alkaline phase. The overall ratio of acidic to alkaline phase in the compositions of the invention, both monolayer and multilayer, is preferably from 10:1 to 1:20, more preferably from 2:1 to 1:5 and especially from 1:1 to 1:3. The multilayer tablets can be prepared by precompressing the pregranulated components of a first layer of the multilayer tablet to a pressure of about $10^5$ kPa in a punch and dye tabletting press, adding the pregranulated components of the neighbouring layer with precompression as appropriate and repeating until the components of all layers of the tablet are in the press. The tablet is then given a final compression at about $1.5.10^5$ kPa. A binder as described above will normally be present in each of the layers of the multilayer tablet.

The solid base material of the compositions of the invention can be supplemented by other usual components of cleansing tablet formulations, especially surfactants, chelating agents, enzymes, flavor oils such as oils of spearmint, peppermint and wintergreen, dyestuffs, sweeteners, foam depressants such as dimethylpolysiloxanes, foam stabilizers such as the fatty acid sugar esters, preservatives, lubricants such as talc, magnesium stearate, finely divided amorphous pyrogenic silicas, etc. The free moisture content of the final composition is desirably less than 1% and especially less than 0.5%.

The surface active agent used in the compositions of the invention can be selected from the many available that are compatible with the other ingredients of the denture cleanser, both in the dry state and in solution. Such materials are believed to improve the effectiveness of the other ingredients of the composition by aiding their penetration into the interdental surfaces. Also, these materials aid in the removal of food debris attached to the teeth. Between 0.1 and 5 percent by weight of the dry composition of a dry powder or granular anionic surface active agent, such as sodium lauryl sulfate, sodium N-lauroylsarcosinate, sodium lauryl sulfoacetate or dioctyl sodium sulfosuccinate or ricinoleyl sodium sulfosuccinate, may, for example, be included in the composition and preferably the surface active agent comprises between 2 and 4 percent of the composition.

Suitable cationic, non-ionic and ampholytic surface active agents include, for example, quaternary ammonium compounds such as cetyltrimethylammonium bromide, condensation products of alkylene oxides such as ethylene or propylene oxide with fatty alcohols, phenols, fatty amines or fatty acid alkanolamides, the fatty acid alkanol amides themselves, esters of long-chained ($C_8$-$C_{22}$) fatty acids with polyalcohols or sugars, for example glycerylmonostearate or saccharosemonolaurate or sorbitolpolyoxyethylenemono-or di-stearate, betaines, sulphobetaines or long-chain alkylaminocarboxylic acids.

Chelating agents beneficially aid cleaning and bleach stability by keeping metal ions, such as calcium, magnesium, and heavy metal cations in solution. Examples of suitable chelating agents include sodium tripolyphosphate, sodium acid pyrophosphate, tetrasodium pyrophosphate, aminopolycarboxylates such as nitrilotriacetic acid and ethylenediamine tetracetic acid and salts thereof, and polyphosphonates and aminopolyphosphonates such as hydroxyethanediphosphonic acid, ethylenediamine tetramethylenephosphonic acid, diethylenetriaminepentamethylenephosphonic acid and salts thereof. The chelating agent selected is not critical except that it must be compatible with the other ingredients of the denture cleanser when in the dry state and in aqueous solution. Advantageously, the chelating agent comprises between 0.1 and 60 percent by weight of the composition and preferably between 0.5 and 30 percent. Phosphonic acid chelating agents, however, preferably comprise from 0.1 to 1 percent, preferably from 0.1% to 0.5% by weight of composition.

Enzymes suitable for use herein are exemplified by proteases, alkalases, amylases, lipases, dextranases, mutanases, glucanases etc. The enzyme is expediently present in a discrete layer of a multilayer tablet.

The following Examples further describe and demonstrate the preferred embodiments within the scope of the present invention.

EXAMPLES I TO IV

The following are representative two-layer denture cleansing tablets according to the invention. The percentages are by weight of the corresponding layer.

|                          | I    | II  | III  | IV   |
|--------------------------|------|-----|------|------|
| **Acidic Layer**         |      |     |      |      |
| Tartaric Acid            | 25   | 22  | 15   | –    |
| Citric Acid              | –    | –   | 15   | 20   |
| Sodium Carbonate         | 4    | 5   | –    | 5    |
| Sulphamic Acid           | 10   | 12  | 5    | 14   |
| PEG 20,000               | 4    | 3   | 1    | 3    |
| PVP 40,000               | –    | –   | 3    | –    |
| Sodium Bicarbonate       | 24.5 | 23  | 26.4 | 37.2 |
| Potassium Persulfate     | 15   | 12  | 16   | 5    |
| Sodium acid Pyrophosphate| 7    | 10  | 8    | 7    |
| Pyrogenic Silica         | 2    | 3   | –    | 1    |
| Talc                     | –    | –   | 2    | –    |
| TAED[1]                  | 7    | –   | 6    | –    |
| TMHOS[2]                 | –    | 8   | –    | 5    |
| EDTMP[3]                 | –    | –   | 1    | 1    |
| Ricinoleylsulfosuccinate | 0.5  | 1   | 0.6  | 0.8  |
| Flavour                  | 1    | 1   | 1    | 1    |

### Alkaline Layer

| | | | | |
|---|---|---|---|---|
| Sodium Perborate Monohydrate | 32 | 30 | 10 | 35 |
| Sodium Bicarbonate | 19 | 21 | 17 | 20 |
| EDTA | 3 | 2 | – | – |
| Sodium Tripolyphosphate | 12 | 10 | – | 8 |
| Sodium Pyrophosphate | – | – | 6 | – |
| PEG 20,000 | 2 | 3 | – | – |
| PEO[4] | – | – | – | 3 |
| Potassium Persulfate | 26 | 28 | 30 | 25 |
| Sodium Carbonate | 2 | 2 | 30 | 5 |
| Magnesium Stearate | – | – | – | 2 |
| Pyrogenic Silica | 2 | 2 | – | – |
| $C_{8-12}$ fatty acid glyceride | – | – | 5 | – |
| Dye/Flavour | 2 | 2 | 2 | 2 |

1  Tetraacetylethylene diamine

2  Sodium 3,5,5-trimethylhexanoyloxybenzene sulfonate

3  Ethylenediaminetetramethylenephosphonic acid

4  Polyoxyethylene – molecular weight 100,000

In Examples I to IV above, the weight ratio of alkaline layer:acidic layer in each instance, is 70:30. The overall tablet weight is 4 g; diameter 25 mm.

The denture cleansing tablets of Examples I to IV display more rapid and efficacious denture cleansing performance and bacterial activity than corresponding compositions which are free of peroxyacid bleach precursor.

## Claims

1. Denture cleansing tablet comprising an inorganic persalt bleaching agent, an organic peroxyacid bleach precursor and a solid base material which comprises at least one alkali metal carbonate or bicarbonate and at least one non-toxic, physiologically-acceptable organic acid and which in the presence of water releases carbon dioxide with effervescence, and wherein the organic peroxyacid bleach precursor is selected from polyacylated alkylenediamines and carboxylic esters having the general formula AcL wherein Ac is the acyl moiety of an organic carboxylic acid comprising an optionally substituted, linear or branched $C_6$-$C_{20}$ alkyl or alkenyl moiety or a $C_6$-$C_{20}$ alkyl-substituted aryl moiety and L is a leaving group, the conjugate acid of which has a pKa in the range from 4 to 13, and wherein are exluded denture cleansing tablets which additionally comprise a polymeric fluorocarbon binder.

2. Cleansing tablet according to Claim 1 wherein the inorganic persalt bleaching agent comprises one or more bleaching agents selected from alkali metal persulfates, alkali metal perborates and mixtures thereof.

EP 0 323 049 B1

**3.** Denture cleansing tablet comprising an inorganic persalt bleaching agent, an organic peroxyacid bleach precursor and a solid base material which comprises at least one alkali metal carbonate or bicarbonate and at least one non-toxic, physiologically-acceptable organic acid and which in the presence of water releases carbon dioxide with effervescence, and wherein the organic peroxyacid bleach precursor is selected from polyacylated alkylenediamines and carboxylic esters having the general formula AcL wherein Ac is the acyl moiety of an organic carboxylic acid comprising an optionally substituted, linear or branched $C_6$-$C_{20}$ alkyl or alkenyl moiety or a $C_6$-$C_{20}$ alkyl-substituted aryl moiety and L is a leaving group, the conjugate acid of which has a pKa in the range from 4 to 13, and wherein further the inorganic bleaching agent comprises an alkali metal persulfate and the solid base material comprises at least one component phase having an acid pH in aqueous medium, said at least one component phase having incorporated therein the organic peroxy acid bleach precursor and at least a portion of the alkali metal persulfate.

**4.** Cleansing tablet according to Claim 2 or 3 wherein the inorganic bleaching agent comprises a mixture of alkali metal persulfate and alkali metal perborate bleaching agents and wherein the solid base material comprises two or more component phases having differng pH in aqueous medium, at least one phase having an acidic pH and having incorporated therein the organic peroxyacid bleach precursor and at least a portion of the alkali metal persulfate, and at least one other phase having an alkaline pH and having incorporated therein the alkali metal perborate.

**5.** Cleansing tablet according to Claim 4 wherein the alkaline phase dissolves or disperses in water more slowly than or later than the acidic phase in order to provide, on placing the tablet in water, an initial pH in the acidic range.

**6.** Cleansing tablet according to Claim 5 wherein the initial pH is from 2 to 6.5, preferably from 3 to 5, and is maintained within the acid range for from 30 seconds to 3 hours, preferably from 1 minute to 60 minutes, more preferably from 2 to 30 minutes.

**7.** Cleansing tablet according to any of Claims 4 to 6 wherein the alkaline phase is present in sufficient excess of the acidic phase in order to shift, upon completion of effervescence, the pH of the aqueous medium into the alkaline range, preferably to a pH of from 7.5 to 9.

**8.** Cleansing tablet according to any of Claims 4 to 7 wherein at least the acidic phase incorporates both the alkali metal carbonate or bicarbonate and the non-toxic physiologically-acceptable organic acid.

**9.** Cleansing tablet according to any of Claims 4 to 8 comprising two or more discrete layers, at least one layer comprising the acid phase and at least one other layer comprising the alkaline phase.

**10.** Use of an organic peroxy acid bleach precursor in the manufacture of a denture cleanser which is efficaceous on plaque, mucilageneous and bacterial deposits, wherein the denture cleanser comprises an inorganic persalt bleaching agent and a solid base material which comprises at least one alkali metal carbonate or bicarbonate and at least one non-toxic, physiologically-acceptable organic acid and which in the presence of water releases carbon dioxide with effervescence, and wherein the organic peroxyacid bleach precursor is selected from polyacylated alkylenediamines and carboxylic esters having the general formula AcL wherein Ac is the acyl moiety of an organic carboxylic acid comprising an optionally substituted, linear or branched $C_6$-$C_{20}$ alkyl or alkenyl moiety or a $C_6$-$C_{20}$ alkyl-substituted aryl moiety and L is a leaving group, the conjugate acid of which has a pKa in the range from 4 to 13, and wherein is excluded use of an organic peroxy acid bleach precursor in the manufacture of a denture cleanser which additionally comprises a polymeric fluorocarbon binder.

**Patentansprüche**

**1.** Gebißreinigungstablette, welche ein anorganisches Persalz-Bleichmittel, einen organischen Peroxysäure-Bleichmittelprecursor und ein festes Grundstoffmaterial enthält, welches letztgenannte wenigstens ein Alkalimetallcarbonat oder -bicarbonat und wenigstens eine nicht-toxische, physiologisch annehmbare organische Säure enthält und in Gegenwart von Wasser Kohlendioxid unter Aufbrausen freisetzt, wobei der organische Peroxysäure-Bleichmittelprecursor aus polyacylierten Alkylendiaminen und Carbonsäureestern mit der allgemeinen Formel AcL ausgewählt ist, worin Ac den Acylrest einer organi-

8

schen Carbonsäure darstellt, welcher einen gegebenenfalls substituierten, linearen oder verzweigten $C_6$-$C_{20}$-Alkyl- oder Alkylenrest oder einen $C_6$-$C_{20}$- Alkyl-substituierten Arylrest umfaßt und L eine Leaving-Gruppe ist, und dessen zugehörige Säure einen $pK_a$-Wert in einem Bereich von 4 bis 13 aufweist, wobei Gebißreinigungstabletten ausgeschlossen sind, welche zusätzlich ein polymeres Fluorkohlenstoff-Bindemittel enthalten.

2. Reinigungstablette nach Anspruch 1, wobei das anorganische Persalz-Bleichmittel ein oder mehrere Bleichmittel umfaßt, welche aus Alkalimetallpersulfaten, Alkalimetallperboraten und Gemischen hievon ausgewählt sind.

3. Gebißreinigungstablette, welche ein anorganisches Persalz-Bleichmittel, einen organischen Peroxysäure-Bleichmittelprecursor und ein festes Grundstoffmaterial enthält, welches letztgenannte wenigstens ein Alkalimetallcarbonat oder -bicarbonat und wenigstens eine nicht-toxische, physiologisch annehmbare organische Säure enthält und in Gegenwart von Wasser Kohlendioxid unter Aufbrausen freisetzt, wobei der organische Peroxysäure-Bleichmittelprecursor aus polyacylierten Alkylendiaminen und Carbonsäureestern mit der allgemeinen Formel AcL ausgewählt ist, worin Ac den Acylrest einer organischen Carbonsäure darstellt, welcher einen gegebenenfalls substituierten, linearen oder verzweigten $C_6$-$C_{20}$-Alkyl- oder -Alkenylrest oder einen $C_6$-$C_{20}$-Alkyl-substituierten Arylrest umfaßt und L eine Leaving-Gruppe ist, und dessen zugehörige Säure einen $pK_a$- Wert in einem Bereich von 4 bis 13 aufweist, und worin weiterhin das anorganische Bleichmittel ein Alkalimetallpersulfat umfaßt, und worin das feste Grundstoffmaterial wenigstens eine Komponentenphase umfaßt, welche in einem wässerigen Medium einen sauren pH-Wert aufweist, und wobei dieser wenigstens einen Komponentenphase der organische Peroxysäure-Bleichmittelprecursorund wenigstens ein Teil des Alkalimetallpersulfates beigemengt sind.

4. Reinigungstablette nach Anspruch 2 oder 3, worin das anorganische Bleichmittel ein Gemisch von Bleichmitteln auf der Basis von Alkalimetallpersulfat und Alkalimetallperborat umfaßt und worin das feste Grundstoffmaterial zwei oder mehrere Komponentenphasen umfaßt, welche in wäßrigem Medium voneinander verschiedene pH-Werte aufweisen, wobei wenigstens eine Phase einen sauren pH-Wert aufweist und als Beimengung darin den organischen Peroxysäure-Bleichmittelprecursor und wenigstens einen Teil des Alkalimetallpersulfates aufweist, und wobei wenigstens eine andere Phase einen alkalischen pH-Wert aufweist und als Beimengung darin das Alkalimetallperborat aufweist.

5. Reinigungstablette nach Anspruch 4, wobei sich die alkalische Phase in Wasser langsamer oder später auflöst oder dispergiert als die saure Phase, um so beim Hineinlegen der Tablette in Wasser einen Anfangs-pH-Wert im sauren Bereich zu schaffen.

6. Reinigungstablette nach Anspruch 5, wobei der Anfangs-pH-Wert 2 bis 6,5, vorzugsweise 3 bis 5 beträgt und während 30 s bis 3 h, vorzugsweise während 1 min bis 60 min, und in höherem Maße bevorzugt während 2 min bis 30 min im sauren Bereich gehalten wird.

7. Reinigungstablette nach einem der Ansprüche 4 bis 6, worin die alkalische Phase in einem ausreichenden Überschuß über die saure Phase vorhanden ist, um den pH-Wert des wäßrigen Mediums nach Beendigung des Aufbrausens in den alkalischen Bereich, vorzugsweise auf einen pH-Wert von 7,5 bis 9, zu verschieben.

8. Reinigungstablette nach einem der Ansprüche 4 bis 7, worin wenigstens der sauren Phase sowohl das Alkalimetallcarbonat oder -bicarbonat als auch die nicht-toxische, physiologisch annehmbare organische Säure beigemengt sind.

9. Reinigungstablette nach einem der Ansprüche 4 bis 8, welche zwei oder mehrere getrennte Schichten aufweist, wobei wenigstens eine Schicht die saure Phase enthält und wobei wenigstens eine andere Schicht die alkalische Phase enthält.

10. Verwendung eines organischen Peroxysäure-Bleichmittelprecursors bei der Herstellung eines Gebißreinigungsmittels, welches gegenüber Zahnbelag, schleimigen und bakteriellen Ablagerungen wirksam ist, wobei das Gebißreinigungsmittel ein anorganisches Persalz-Bleichmittel und ein festes Grundstoffmaterial enthält, welches letztgenannte wenigstens ein Alkalimetallcarbonat oder -bicarbonat und wenigstens eine nichttoxische, physiologisch annehmbare organische Säure enthält und in Gegenwart von Wasser Kohlendioxid unter Aufbrausen freisetzt, wobei der organische Peroxysäure-Bleichmittelprecursor aus polyacylierten Alkylendiaminen und Carbonsäureestern mit der allgemeinen Formel AcL ausgewählt ist, worin Ac den Acylrest einer organischen Carbonsäure darstellt, welcher einen gegebenenfalls substituierten, linearen oder verzweigten $C_6$-$C_{20}$-Alkyl- oder Alkenylrest oder einen $C_6$-$C_{20}$-Alkyl-substituierten Arylrest umfaßt und L eine Leaving-Gruppe ist, und dessen zugehörige Säure einen $pK_a$-Wert in einem Bereich von 4 bis 13 aufweist, und wobei die Verwendung eines organischen Peroxysäure-Bleichmittelprecursors bei der Herstellung eines Gebißreinigungsmittels, welches zusätzlich ein polymeres Fluorkohlenstoff-Bindemittel enthält, ausgeschlossen ist.

**Revendications**

1. Comprimé de nettoyage pour dentiers comprenant un agent de blanchiment persel minéral, un précurseur de blanchiment de peroxyacide organique et une matière de base solide qui comprend au moins un carbonate ou un bicarbonate de métal alcalin et au moins un acide organique non toxique physiologiquement acceptable et qui en présence d'eau libère du gaz carbonique avec effervescence, et dans lequel le précurseur de blanchiment de peroxyacide organique est choisi parmi les alkylènediamines polyacylées et les esters carboxyliques ayant la formule générale AcL dans laquelle Ac est la fraction acyle de l'acide organique carboxylique comprenant une fraction alkyle ou alcényle en $C_6$ à $C_{20}$ linéaire ou ramifiée éventuellement substituée, ou une fraction aryle substituée par un alkyle en $C_6$ à $C_{20}$ et L est un groupe quittant, dont l'acide conjugué a un pKa dans la gamme de 4 à 13, et dans lequel sont exclus les comprimés de nettoyage pour dentiers qui comprennent de plus un liant polymère fluorocarboné.

2. Comprimé de nettoyage selon la revendication 1, dans lequel l'agent de blanchiment de persel minéral comprend un ou plusieurs agents de blanchiment choisis parmi les persulfates de métal alcalin, les perborates de métal alcalin et leurs mélanges.

3. Comprimé de nettoyage pour dentiers comprenant un agent de blanchiment de persel minéral, un précurseur de blanchiment de peroxyacide organique et une matière de base solide qui comprend au moins un carbonate ou un bicarbonate de métal alcalin et au moins un acide organique non toxique physiologiquement acceptable et qui, en présence d'eau, libère du gaz carbonique avec effervescence, et dans lequel le précurseur de blanchiment de peroxyacide organique est choisi parmi les alkylènediamines polyacylées et les esters carboxyliques ayant la formule générale AcL dans laquelle Ac est la fraction acyle d'un acide organique carboxylique comprenant une fraction alkyle ou alcényle en $C_6$ à $C_{20}$ linéaire ou ramifiée, éventuellement substituée, ou une fraction aryle substituée par un groupe alkyle en $C_6$ à $C_{20}$ et L est un groupe quittant, dont l'acide conjugué a un pKa dans la gamme de 4 à 13, et dans lequel en outre l'agent de blanchiment minéral comprend un persulfate de métal alcalin et la matière de base solide comprend au moins une phase de composant ayant un pH dans un milieu aqueux, ladite au moins une phase de composant incorporant le précurseur de blanchiment peroxyacide organique et au moins une partie de persulfate de métal alcalin.

4. Comprimé de nettoyage selon la revendication 2 ou 3, dans lequel l'agent de blanchiment minéral comprend un mélange de persulfate de métal alcalin et des agents de blanchiment de perborate de métal alcalin et dans lequel la matière de base solide comprend deux ou plusieurs phases de composants ayant différents pH en milieu aqueux, au moins une phase ayant un pH acide et incorporant le précurseur de blanchiment de peroxyacide organique et au moins une partie de persulfate de métal alcalin, et au moins une autre phase ayant un pH basique et incorporant le perborate de métal alcalin.

5. Comprimé de nettoyage selon la revendication 4, dans lequel la phase basique se dissout ou se disperse dans l'eau plus lentement que ou plus tard que la phase acide afin de créer, en mettant le comprimé dans l'eau, un pH initial dans la plage acide.

6. Comprimé de nettoyage selon la revendication 5, dans lequel le pH initial est d'environ 2 à 6,5 de préférence de 3 à 5, et est maintenu dans la plage acide de 30 secondes à 3 heures, de préférence de 1 minute à 60 minutes, plus particulièrement préféré de 2 à 30 minutes.

**7.** Comprimé de nettoyage selon l'une quelconque des revendications 4 à 6, dans lequel la phase basique est présente dans un excès suffisant de phase acide afin de décaler, après achèvement de l'effervescence, le pH du milieu aqueux dans la plage basique, de préférence à un pH de 7,5 à 9.

**8.** Comprimé de nettoyage selon l'une quelconque des revendications 4 à 7, dans lequel au moins la phase acide incorpore à la fois le carbonate ou le bicarbonate de métal alcalin et l'acide organique non toxique physiologiquement acceptable.

**9.** Comprimé de nettoyage selon l'une quelconque des revendications 4 à 8 comprenant deux ou plusieurs couches discrètes, au moins une couche comprenant la phase acide et au moins une autre couche comprenant le phase basique.

**10.** Utilisation d'un précurseur de blanchiment de peroxyacide organique dans la fabrication d'un agent de nettoyage pour dentiers qui est efficace sur la plaque dentaire, les dépôts mucilagineux et bactériens, dans lequel l'agent de nettoyage pour dentiers comprend un agent de blanchiment de persel minéral et une matière de base solide qui comprend au moins un carbonate ou un bicarbonate de métal alcalin et au moins un acide organique non toxique physiologiquement acceptable et qui, en présence d'eau, libère du gaz carbonique avec effervescence, et dans lequel le précurseur de blanchiment peroxyacide organique est choisi parmi les alkylènediamines polyacylées et les esters carboxyliques ayant la formule générale AcL avec laquelle Ac est la fraction acyle d'un acide organique carboxylique comprenant une fraction alcényle ou alkyle en $C_6$ à $C_{20}$ linéaire ou ramifiée éventuellement substituée ou une fraction aryle substituée par un groupe alkyle en $C_6$ à $C_{20}$ et L est un groupe quittant, dont l'acide conjugué a un pKa dans la gamme de 4 à 13, et dans lequel est exclue l'utilisation d'un précurseur de blanchiment de peroxyacide organique dans la fabrication d'un agent de nettoyage pour dentiers qui comprend de plus un liant polymère fluorocarboné.